# FASCICULE DE BREVET EUROPEEN

(11) **EP 0 461 237 B1**
(45) Date de publication et mention de la délivrance du brevet: **09.08.1995**
(21) Numéro de dépôt: 91902299.6
(22) Date de dépôt: 27.12.1990
(51) Int. Cl.: C07C 211/42, A61K 31/13, A61K 31/165, A61K 31/395

(54) **UTILISATION DE DERIVES DU 9,10-DIHIDROPHENANTHRENE POUR LA PREPARATION D'UN MEDICAMENT ANTI-TUMORAL ET NOUVEAUX DERIVES**
VERWENDUNG VON DERIVATEN VON 9,10-DIHYDROPHENANTHREN ZUR HERSTELLUNG EINES ANTITUMORALEN MEDIKAMENTES UND NEUEN DERIVATEN DARAUS
USE OF 9,10-DIHYDROPHENANTHRENE DERIVATIVES TO PREPARE AN ANTITUMORAL DRUG, AND NOVEL DERIVATIVES THEREOF

(30) Priorité: 28.12.1989 FR 8917302
(43) Date de publication de la demande: 18.12.1991
(73) Titulaire: ROUSSEL UCLAF, 93230 Romainville (FR)
(72) Inventeur: CZECH, Jörg, D-3550 Marburg (DE); SEDLACEK, Hans, Harald, D-3550 Marburg (DE); NEDELEC, Lucien, F-93340 Le Raincy (FR); GUILLAUME, Jacques, F-75020 Paris (FR); MARCHANDEAU, Christian, F-77410 Annet s/Marne (FR)
(74) Mandataire: Vieillefosse, Jean-Claude
(86) Numéro de dépôt international: FR9000952
(87) Numéro de publication internationale: WO9109833

(56) Documents cités:
- US-A- 4 511 582
- J. Chem. Soc. Perkin Trans. II, no. 5, mai 1988, (Londres, GB), M. Yasuda et al.: "Photochemical reactions of aromatic compounds. Part 44. Mechanisms for direct photoamination of arenes with ammonia and amines in the presence of m-dicyanobenzene", pages 745-751
- Journal of Medicinal Chemistry, vol. 18, no. 1, janvier 1975, (Washington, DC, US), J.G. Cannon et al.: "Centrally acting emetics. 8. Conformational aspects of certain dihydrophenanthrene congeners of apomorphine", pages 108-110
- Journal of Medicinal Chemistry, vol. 21, no. 4, avril 1978, American Chemical Society, (Washington, DC, US), D.E. Nichols et al.: "2,3,Dihydroxy-9-amino-9,10-dihydrophenanthrene, a rigid congener of dopamine and isoapomorphine", pages 395-398
- J. Org. Chem., vol. 52, no. 5, 6 mars 1987, American Chemical Society, (Washington, DC, US), M. Yasuda et al.: "Direct photo-amination of arenes with ammonia and primary amines in the presence of electron acceptors", pages 753-759
- Journal of Computer-Aided Molecular Design, no. 1, 1987, ESCOM Science Publishers B.V., (Leiden, NL), I. Pettersson et al.: "Structure-activity relationships for apomorphine congeners. Conformational energies vs. biological activities", pages 143-152
- British Journal of Pharmacology, vol. 67, no. 3, 1979, (Londres, GB), J.B. Freedman et al.: "Effects of dopamine receptor agonists and antagonists in the rat nucleus accumbens", pages 430P-431P
- J. Pharm. Pharmacol., vol. 35, no. 12, decembre 1983, D. Kocjan et al.: "Conformationally restricted dopamine congeners a molecular mechanics-based study", pages 780-785

## Description

L'invention concerne l'utilisation de dérivés du 9,10-dihydrophénanthrène pour la préparation d'un médicament anti-tumoral et notamment d'un médicament anti-cancéreux, l'application à titre de médicaments de dérivés du 9,10-dihydrophénanthrène et des produits dérivés de cette structure.

L'expression d'oncogènes dans une cellule de mammifère conduit à la transformation de types de cellules normales en cellules cancéreuses. Ladite transformation est provoquée par l'infection d'une cellule par un retrovirus. On peut citer comme exemple bien connu, l'infection des poules par le virus de Rous qui conduit à l'apparition d'un cancer. L'oncogène correspondant qui est responsable de la transformation maligne a été dénommé gène "SRC" (J.S. Grugge RL Erikson, Nature 269, 346-348 (1977).

Beaucoup des oncogènes connus jusqu'alors, sont caractérisés par l'expression d'une protéine possédant une activité de kinase. Ces enzymes catalysent le transfert du groupe phosphate terminal de l'ATP sur un aminoacide. Contrairement à beaucoup d'autres protéinekinases qui transfèrent le groupe phosphate sur un reste séryle ou thréonyle, la plupart des kinases oncogènes phosphorylent un reste tyrosyle de la chaîne protéinique. Par ailleurs, il est connu que les produits d'oncogènes à savoir ceux des oncogènes v-mos, v-mil et v-raf possèdent une activité protéinekinase sérine/thréonine spécifique (K. Rölling et al., Nature (London) 312, 558-561 (1984), B. Singh et al., Journal of Virology 60, 1149-1152 (1986).

L'activité tyrosinekinase fait partie intégrante de la fonction de certains récepteurs de facteurs de croissance. De nouveaux résultats montrent que la croissance de beaucoup de tumeurs dépend de la présence de facteurs de croissance tels que l'Epidermal Growth Factor (EGF) le "Transforming Growth Factor Alpha (TGFAlpha) ou le "Platelet Derived Growth Factor (PDGF) (A.S. Goustin, G. D. Shipley, H.L. Moses, Cancer Research 46, 1015-1029 (1986). Comme conséquence de la liaisor du facteur de croissance avec son récepteur, la tyrosinekinase qui est un composant propre du récepteur du facteur de croissance, est stimulée.

On peut donc s'attendre à ce qu'un inhibiteur de la tyrosinekinase et également de la sérine/thréoninekinase puisse inhiber la croissance et la prolifération tumorales et puisse être utilisé dans la thérapie anti-tumorale.

Or il vient d'être découvert de façon surprenante que les produits de formule (I) telle que définie ci-après sont des inhibiteurs de kinases oncogènes telles que la tyrosinekinase, la sérine/thréoninekinase et la tyrosinekinase du récepteur du facteur de croissance et sont donc utilisables pour le traitement de maladies tumorales. Les produits de formule (I) qui présentent des propriétés anti-prolifératives, anti-oncotiques et carcinostatiques peuvent en particulier, être utilisés pour inhiber la croissance et la prolifération tumorales et dans la thérapie tumorale.

Le document US-P-4,511,582 décrit des dérivés du 2-méthyl-2-amino-1,3-propanediol contenant le groupe phénanthylméthyl-, qui sont utilisables dans la thérapie anti-cancer.

La présente invention a donc pour objet l'utilisation des produits de formule générale (I) : 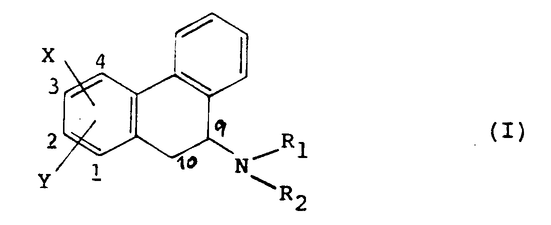
dans laquelle X et Y, identiques ou différents sont choisis parmi les atomes d'hydrogène et les radicaux hydroxy et alkyloxy ayant de 1 à 4 atomes de carbone et R₁ et R₂ identiques ou différents sont choisis parmi les atomes d'hydrogène, les radicaux alkyle, alkényle ou alkynyle ayant au plus 4 atomes de carbone, les radicaux arylalkyle et acyle, ou bien R₁ et R₂ forment ensemble avec l'atome d'azote auquel ils sont liés un hétérocycle à cinq ou six chaînons pouvant comporter un second hétéroatome choisi parmi les atomes d'azote, d'oxygène ou de soufre, éventuellement substitué ainsi que leurs sels d'addition avec les acides minéraux ou organiques pour la préparation d'un médicament anti-tumoral.

Parmi les valeurs de X et Y on peut citer outre les valeurs hydrogène et hydroxy, les radicaux méthoxy, éthoxy, propyloxy, isopropyloxy, butyloxy, sec-butyloxy, isobutyloxy, tert-butyloxy. On préfère la valeur hydroxy.

Parmi les valeurs de R₁ et R₂, on peut citer outre la valeur hydrogène, les radicaux suivants :
- méthyle, éthyle, propyle, isopropyle, butyle, isobutyle, sec-butyle, tert-butyle,
- vinyle, allyle, propényle,
- éthynyle, propargyle, 1-propynyle, 3-butynyle,
- benzyle, phényléthyle,
- formyle, acétyle, propionyle, benzoyle.
R₁ et R₂ peuvent également former avec l'atome d'azote auquel ils sont liés les radicaux pyrrolidinyle, imidazolinyle, imidazolidinyle, pyrazolidinyle, pipéridinyle, morpholinyle, pipérazinyle, méthylpipérazinyle, éthylpipérazinyle ou propylpipérazinyle.

Les sels d'addition avec les acides minéraux ou organiques peuvent être, par exemple, les sels formés avec les acides chlorhydrique, bromhydrique, nitrique, sulfurique, phosphorique, acétique, formique, propionique, malonique, maléique, fumarique, succinique, tartrique, citrique, oxalique, glyoxylique, aspartique, alcanesulfoniques tels que les acides méthane ou éthanesulfoniques, arènesulfoniques, tels que les acides benzène ou paratoluène sulfoniques et arylcarboxyliques, tels que benzoïques. Les sels préférés sont les chlorhydrates, bromhydrates et acétates.

Les médicaments objets de la présente invention peuvent se présenter sous la forme de compositions pharmaceutiques destinées à l'administration par voie digestive, parentérale ou locale. Ils peuvent être prescrits sous forme de comprimés simples ou dragéifiés, de gélules, de granulés, de suppositoires, de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de 5 cacao, les véhicules aqueux ou non, les corps gras d'origine animale ou végétale, les dérivés paraffiniques, les glycols, les divers agents mouillants, dispersants ou émulsifiants, les conservateurs.

La posologie varie en fonction de l'affection à traiter et de la voie d'administration, elle peut varier par exemple de 10 à 500 mg par jour chez l'adulte par voie orale. Préférentiellement la dose utilisable peut être de 50 à 250 mg par jour par voie orale.

L'invention a particulièrement pour objet l'utilisation des produits de formule générale (Iₐ) : 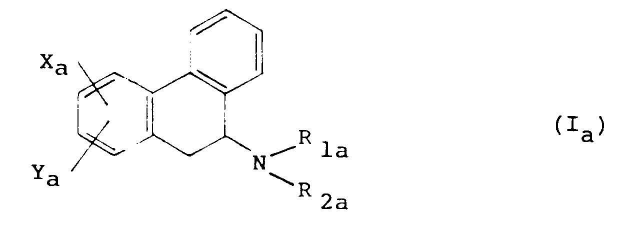
dans laquelle Xₐ et Yₐ sont tels que soit ils représentent chacun un radical hydroxy, soit l'un représente un radical hydroxy et l'autre un atome d'hydrogène,
R₁ₐ et R₂ₐ sont choisis parmi, l'hydrogène, les radicaux alkyle ayant de 1 à 4 atomes de carbone, de préférence le méthyle et les radicaux acyle, de préférence formyle, acétyle ou benzoyle, étant entendu qu'ils ne peuvent pas être tous les deux hydrogène,
ainsi que leurs sels d'addition avec les acides minéraux ou organiques, de préférence les chlorhydrates, bromhydrates et acétates, pour la préparation d'un médicament anti-tumoral.

L'invention a plus particulièrement pour objet l'utilisation de l'un des produits suivants :
- 3,4-dihydroxy 9-méthylamino 9,10-dihydrophénanthrène,
- 3,4-dihydroxy 9-diméthylamino 9,10-dihydrophénanthrène, et leurs sels d'addition avec les acides minéraux ou organiques, pour la préparation d'un médicament anti-tumoral.

L'invention a également pour objet à titre de médicaments, les produits de formule générale (I') : 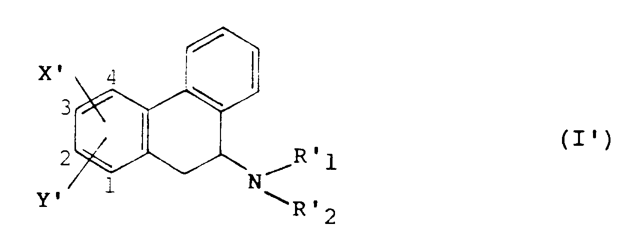
dans laquelle X' et Y', identiques ou différents sont choisis parmi les atomes d'hydrogènes et les radicaux hydroxy et alkyloxy ayant de 1 à 4 atomes de carbone et R'₁ et R'₂ identiques ou différents sont choisis parmi les atomes d'hydrogène, les radicaux alkyle, alkényle ou alkynyle ayant au plus 4 atomes de carbone, les radicaux arylalkyle et acyle, ou bien R'₁ et R'₂ forment ensemble avec l'atome d'azote auquel ils sont liés un hétérocycle à cinq ou six chaînons pouvant comporter un second hétéroatome choisi parmi les atomes d'azote, d'oxygène ou de soufre, éventuellement substitué, étant entendu que :
a) lorsque X' et Y' représentent chacun un radical hydroxy en positions 3 et 4, R'₁ et R'₂ ne peuvent pas représenter chacun un radical méthyle,
b) lorsque X' et Y' représentent chacun un radical hydroxy ou méthoxy en position 2 et 3, R'₁ et R'₂ ne peuvent pas représenter chacun un atome d'hydrogène,
c) lorsque X' et Y' représentent chacun un radical méthoxy en position 2 et 3, R'₁ et R'₂ ne peuvent pas représenter l'un un atome d'hydrogène et l'autre un radical benzoyle, ainsi que les sels d'addition avec les acides minéraux ou organiques.

L'invention a particulièrement pour objet à titre de médicament, le 3,4-dihydroxy 9-méthylamino 9,10-dihydrophénanthrène et ses sels d'addition avec les acides minéraux ou organiques.

L'invention a également pour objet les compositions pharmaceutiques contenant à titre de principe actif l'un au moins des médicaments tels que définis ci-dessus.

Les compositions pharmaceutiques objet de l'invention peuvent être préparées comme indiqué ci-dessus.

L'invention a également pour objet les produits de formule générale (I'') : 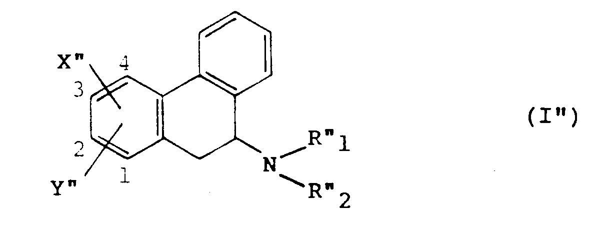
dans laquelle X'' et Y'', identiques ou différents sont choisis parmi les atomes d'hydrogène et les radicaux hydroxy et alkyloxy ayant de 1 à 4 atomes de carbone et R''₁ et R''₂ identiques ou différents sont choisis parmi les atomes d'hydrogène, les radicaux alkyle, alkényle ou alkynyle ayant au plus 4 atomes de carbone, les radicaux arylalkyle et acyle, ou bien
R''₁ et R''₂ forment ensemble avec l'atome d'azote auquel ils sont liés un hétérocycle à cinq ou six chaînons pouvant comporter un second hétéroatome choisi parmi les atomes d'azote, d'oxygène ou de soufre, éventuellement substitué, étant entendu que :
a) lorsque X'' et Y'' représentent chacun un radical hydroxyle ou méthoxy en positions 3 et 4, R''₁ et R''₂ ne peuvent pas représenter chacun un radical méthyle,
b) lorsque X'' et Y'' représentent chacun un radical méthoxy en positions 3 et 4, R''₁ et R''₂ ne peuvent pas représenter chacun un atome d'hydrogène,
c) lorsque X'' et Y'' représentent chacun un radical hydroxy ou méthoxy en positions 2 et 3, R''₁ et R''₂ ne peuvent pas représenter chacun un atome d'hydrogène,
d) lorsque X'' et Y'' représentent chacun un radical méthoxy en positions 2 et 3, R''₁ et R''₂ ne peuvent pas représenter l'un un atome d'hydrogène et l'autre un radical benzoyle,
e) lorsque X'' et Y'' représentent chacun un atome d'hydrogène R''₁ et R''₂ sont tels que :
   i) R''₁ et R''₂ ne peuvent pas représenter chacun un atome d'hydrogène ou un radical méthyle ou éthyle,
   ii) l'un de R''₁ et R''₂ ne peut pas représenter un atome d'hydrogène lorsque l'autre représente un radical méthyle, éthyle, allyle, n-propyle, isopropyle, tert-butyle, benzyle ou phényléthyle.
ainsi que leurs sels d'addition avec les acides minéraux ou organiques.

L'invention a plus particulièrement pour objet le produit de formule (I'') telle que définie ci-dessus et répondant à la formule suivante :
- 3,4-dihydroxy 9-méthylamino 9,10-dihydrophénanthrène et ses sels d'addition avec les acides minéraux ou organiques.

Les produits de formule (I'') sont des produits nouveaux. Les produits de formules (I) et (I') connus peuvent être préparés par des procédés décrits dans la littérature.

On peut notamment citer les références suivantes :
- J. Chem. Soc. Perkin Trans. II(5), (1988), 745-751.
- J. Comput-Aided Mol. Des. (1987) 1(2), 143-152
- J. Med. Chem. (1975), 18(1), 108-110
- J. Med. Chem. (1978), 21(4), 395-8
- Br. J. Pharmacol. (1979), 67(3), 430P-431P
- J. Org. Chem. (1987), 52(5), 753-9
- J. Org. Chem. (1985), 50(19), 3667-9
- J. Pharm. Pharmacol. (1983), 35(12), 780-5
- Zh. Org. Khim, (1983), 19(7), 1552-3
On indique ci-après une méthode de préparation des produits de formule (I'') qui fait partie de l'objet de l'invention.

L'invention a aussi pour objet un procédé de préparation des produits de formule (I'') telle que définie ci-dessus caractérisé en ce que l'on fait agir, sur un produit de formule (I''ₐ) : 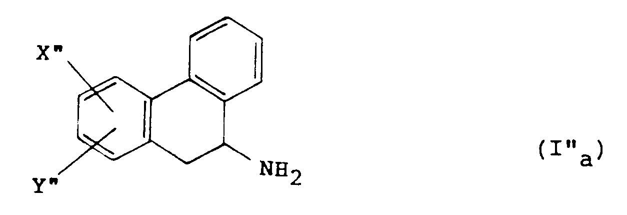
dans laquelle X'' et Y'' ont la signification indiquée ci-dessus,
soit pour préparer un produit de formule (I''_{b}) correspondant à un produit de formule (I'') dans laquelle l'un de R''₁ et R''₂ est différent d'hydrogène et l'autre représente un atome d'hydrogène,
ou bien un équivalent d'un produit de formule R₁₂Hal dans laquelle R₁₂ représente le radical R''₁ ou R''₂ et Hal représente un atome d'halogène,
ou bien un équivalent d'un produit de formule 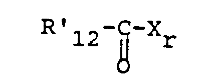
dans laquelle R'₁₂ représente le radical R''₁ ou R''₂ dans lequel on a retranché un atome de carbone et Xᵣ représente un groupement réactif, et soumet éventuellement le produit obtenu à une réduction,
soit pour préparer un produit de formule (I''_{c}) correspondant à un produit de formule (I'') dans laquelle R''₁ et R''₂ sont identiques et ne représentent pas un atome d'hydrogène,
ou bien deux équivalents d'un produit de formule R₁₂Hal dans laquelle R₁₂ et Hal ont la signification précédente,
ou bien deux équivalents d'un produit de formule 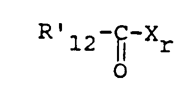
dans laquelle R'₁₂ et Xᵣ ont la signification précédente et soumet éventuellement le produit obtenu à une réduction,
soit pour préparer un produit de formule (I''_{d}) correspondant à un produit de formule (I'') dans laquelle R''₁ et R''₂ sont différents et ne représentent pas un atome d'hydrogène,
ou bien un équivalent d'un produit de formule R₁Hal suivi d'un équivalent d'un produit de formule R₂Hal,
ou bien un équivalent d'un produit de formule 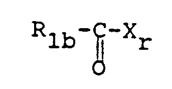
dans laquelle R_{1b} représente le radical R''₁ dans lequel on a retranché un atome de carbone suivi soit d'un équivalent d'un produit de formule R₂Hal, soit d'un équivalent d'un produit de formule 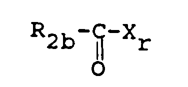
dans laquelle R_{2b} représente le radical R''₂ dans lequel on a retranché un atome de carbone et que dans les deux cas on soumet éventuellement le produit obtenu à une réduction,
soit pour préparer un produit de formule (I''ₑ) correspondant à un produit de formule (I'') dans laquelle R''₁ et R''₂ forment ensemble avec l'atome d'azote auquel ils sont liés un hétérocycle à cinq ou six chaînons pouvant comporter un second hétéroatome choisi parmi les atomes d'azote, d'oxygène ou de soufre éventuellement substitué,
ou bien un produit de formule :

Hal-(CH₂)ₘ-A-(CH₂)ₙ-Hal

dans laquelle m et n sont des entiers différents de zéro choisis de telle façon que leur somme soit égale à 3 ou 4 et A représente un radical méthylène, un atome de soufre, d'oxygène ou d'azote éventuellement substitué,
ou bien un produit de formule : 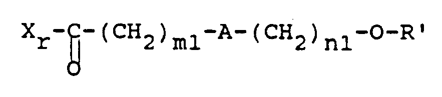
dans laquelle Xᵣ et A ont la signification précédente, m1 et n1 sont des entiers différents de zéro choisis de telle façon que leur somme soit égale à 2 ou 3 et R' représente un groupement protecteur du radical hydroxyle, soumet le produit obtenu à une réaction de déprotection dudit radical hydroxyle puis transforme ledit radical hydroxyle, ou bien en un atome d'halogène ou bien en un dérivé réactif puis soumet l'un ou l'autre des produits obtenus à une réaction de cyclisation et enfin à une réaction de réduction,
et si désiré transforme par hydrolyse les produits de formule (I'') obtenus dans lesquels l'un au moins de X'' ou Y'' représente un radical alkyloxy en produit correspondant dans lequel X'' et Y'' représentent un radical hydroxy et si désiré transforme les produits de formule (I'') en leurs sels d'acides minéraux ou organiques.

Dans un mode préféré d'exécution du procédé ci-dessus, l'atome d'halogène que peut représenter Hal est de préférence un atome de brome, mais Hal peut également représenter un atome de chlore ou d'iode.

L'addition du produit de formule R₁₂Hal sur le produit de formule (I''ₐ) ainsi que l'addition des produits halogénés équivalents (R''₁Hal ou R''₂Hal) est effectuée dans les conditions usuelles, c'est ainsi qu'on opère de préférence en présence d'une base telle que la soude, la potasse ou un sel basique tel que le carbonate de sodium.

L'addition du produit de formule 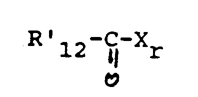
ou des pro duits équivalents 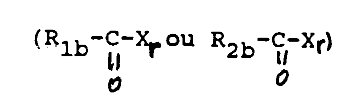
s'effectue également dans les conditions usuelles d'amidification. Le groupement réactif que représente Xᵣ peut être un atome d'halogène ou un reste d'un groupement acyle de manière à ce que 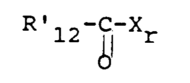
représente un anhydride symétrique ou mixte ou un ester activé. C'est ainsi par exemple que l'on peut utiliser l'anhydride mixte formylacétique. On peut également utiliser les méthodes connues d'amidification telle que l'addition d'un ester sur l'amine en présence de trialkylaluminium. La réduction que l'on effectue éventuellement sur les produits obtenus est également effectuée dans les conditions usuelles. On utilise par exemple un hydrure tel que l'hydrure de lithium aluminium ou un borane tel que le complexe (BH₃, Me₂S). On peut également, lorsque R₁₂ représente un atome d'hydrogène, opérer en présence d'acide formique pour obtenir directement le produit de formule (I''_{b}) dans laquelle l'un de R''₁ ou R''₂ représente un radical méthyle.

Lorsque l'on ajoute un produit de formule : 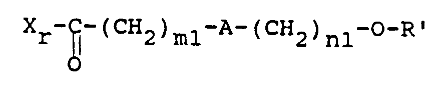
le groupement protecteur que représente R' peut être par exemple un radical tétrahydropyrannyle ou un radical tertbutyle, triméthylsilyle ou un autre trialkylsilyle.

L'élimination de ce groupement est effectué dans les conditions usuelles telles que l'hydrolyse acide à l'aide d'un acide minéral ou organique tel que l'acide chlorhydrique ou l'acide sulfurique.

La réaction d'échange du radical hydroxyle par un atome d'halogène est effectuée également dans les conditions usuelles telle que l'action du chlorure de thionyle. Le dérivé réactif de l'hydroxyle que l'on peut utiliser peut être le tosylate ou le mésylate préparés de façon habituelle. La réaction de cyclisation est effectuée dans les conditions indiquées ci-dessus, pour l'addition des halogénures tels que R₁₂Hal.

L'hydrolyse des substituants X'' et/ou Y'' lorsque l'un au moins de ces substituants représente un radical alkyloxy est effectuée de préférence en milieu acide. On utilise par exemple l'acide chlorhydrique ou de préférence l'acide bromhydrique.

La salification des produits de formule (I'') est effectuée selon les méthodes usuelles. On opère alors de préférence avec l'un des acides cités ci-dessus.

Les produits de formule (I''ₐ) peuvent être préparés comme suit :
on estérifie par les méthodes usuelles un acide de formule (II) : 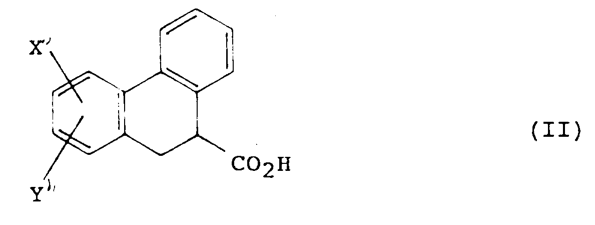
dans laquelle X'' et Y'' ont la signification indiquée ci-dessus de manière à obtenir un produit de formule (III) : 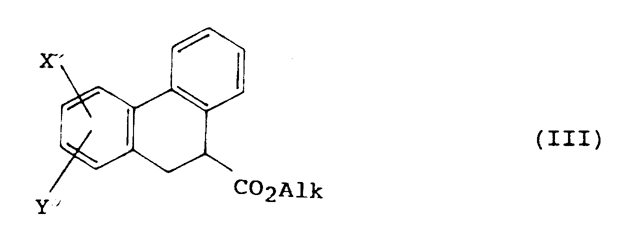
dans laquelle Alk représente un radical alkyle ayant de 1 à 4 atomes de carbone, produit sur lequel on fait agir l'hydrazine pour obtenir le produit de formule (IV) : 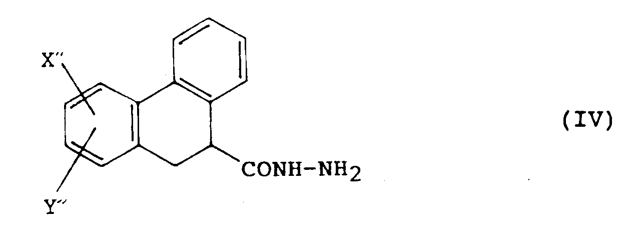
que l'on traite par un nitrite puis par un alcanol pour obtenir le produit de formule (V) : 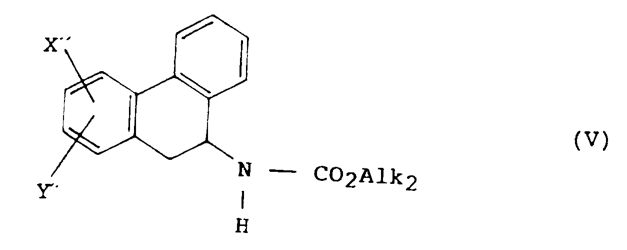
dans laquelle Alk₂ représente un radical alkyle ayant de 1 à 4 atomes de carbone, produit que l'on traite par une base pour obtenir le produit de formule (I''ₐ) attendu.

Dans un mode préféré d'exécution de ce procédé :
a) X'' et Y'' ne représentent pas un radical OH libre mais plutôt un atome d'hydrogène ou un radical alkoxy,
b) l'estérification est réalisée soit par un diazoalcane tel que le diazométhane ou en présence d'un alcanol tel que l'éthanol,
c) la formation de l'hydrazine de formule (IV) est réalisée à l'aide d'hydrate d'hydrazine dans l'éthanol au reflux,
d) le passage des produits de formule (IV) aux produits de formule (V) est réalisé selon la technique décrite dans la référence J. Am. Chem. Soc. (1947) 69, 1998. On utilise une solution aqueuse de nitrite de sodium dans l'acide acétique pour obtenir l'acylazide que l'on transforme in situ, sous l'action d'un alcanol tel que l'éthanol au reflux, en produit attendu.
   Alk₂ représente de préférence un radical éthyle.
e) la saponification des produits de formule (V) en produits de formule (I''ₐ) est réalisée de préférence par la potasse ou la soude alcoolique. On peut ensuite procéder à une acidification par exemple par un acide halohydrique tel que l'acide chlorhydrique ou bromhydrique pour obtenir le sel, chlorhydrate ou bromhydrate correspondant. On trouve une telle réaction dans J. Med. Chem. (1975), 18(1), 108-10.

Les produits de formule (II) peuvent eux-mêmes être préparés comme suit :
on fait agir l'acide phénylacétique ou un sel de cet acide sur un produit de formule (VII) : 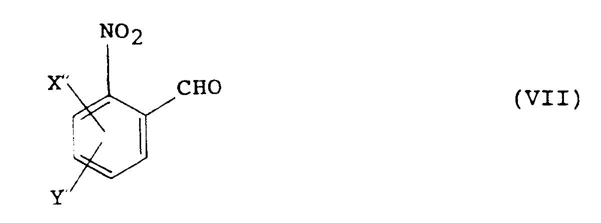
pour obtenir un produit de formule (VIII) : 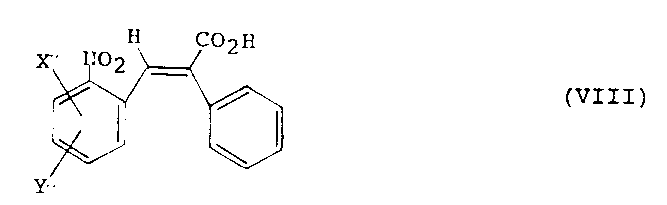
que l'on soumet à une réduction pour obtenir un produit de formule (IX) : 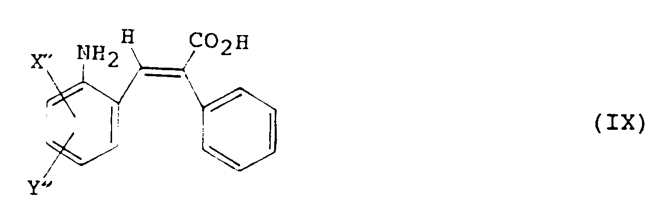
que l'on soumet à un agent de cyclisation pour obtenir un produit de formule (X) : 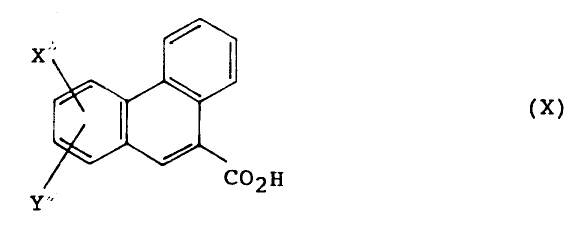
que l'on soumet à un agent de réduction pour obtenir le produit de formule (II) attendu.

Dans un mode préféré d'exécution de cette préparation :
a) on utilise de préférence un sel de l'acide phénylacétique tel que le sel de sodium. La réaction est effectuée en présence d'anhydride acétique,
b) la réduction du produit de formule (VIII) en produit de formule (IX) est effectuée en présence de sulfate ferreux dans l'eau ammoniaquée en chauffant (Ber. 33, 1810, (1900), J. Am. Chem. Soc. 69, 1998, (1947)),
c) la cyclisation des produits de formule (IX) est réalisée dans l'acide sulfurique dilué en présence de nitrite de sodium puis de cuivre préparé extemporanément à partir de sulfate de cuivre,
d) la réduction finale est effectuée de préférence par l'amalgame de sodium à 4 % environ sur le sel de sodium du produit de formule (X) en solution dans la soude aqueuse et on acidifie ensuite à l'acide chlorhydrique (Helv. Chem. Acta 31, 1119-1132, (1948)),
e) la chaîne réactionnelle est effectuée de préférence sur un produit de formule (VII) dans laquelle X'' et Y'' ne représentent pas un radical hydroxy, c'est-à-dire soit un atome d'hydrogène soit un radical alkoxy.

Les nitrobenzaldéhydes de formule (VII) peuvent à leur tour être préparés comme suit :
on effectue une nitration, de préférence à l'aide d'acide nitrique par exemple dans le tétrachlorure de carbone sur un produit de formule (XII) : 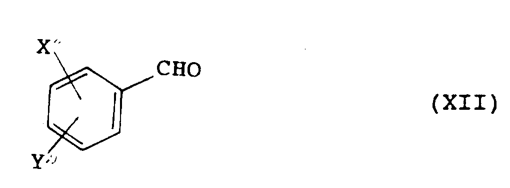
On peut partir par exemple de produits dans lesquels X'' et Y'' sont sous forme protégée telle qu'un radical hydroxy acylé par exemple par un radical acétyle.

On trouvera dans la littérature de nombreuses références décrivant la préparation de produits de formule (VII) par exemple :
- J. Med. Chem. (1987), vol. 30(2) p. 303-18
- J. Med. Chem. (1974), vol. 17(10) p. 1086-90
- Can. J. Chem. (1978), vol. 56(21) p. 2725-30
- J. Org. Chem. (1984), vol. 49(7) p. 1238-46
- EP 0.173.349 (brevet européen)
- J. Med. Chem. (1987), vol. 30(2) p. 295-303
- DE 2.905.054 (brevet allemand)
- Chim. Ther. (1970), vol. 5(4) p. 274-8
- J. Heterocycl. Chem. (1973), vol. 10(4) p. 649-54
- J. Heterocycl. Chem. (1986), vol. 23(6) p. 1805-14
- EP 0.028.473
- Acta. Pharm. Suec, (1979), vol. 16(1) p. 64-73
- J. Indian. Chem. Soc. (1969), vol. 46(7) p. 651-5
- USP 4.044.134 (brevet américain)
- Ann. Chim. (Rome), (1970), vol. 60(10-11) p. 688-96
- Synth. Commun. (1986), vol. 16(6) p. 681-7
- Tétrahedron, (1978), vol. 34(15) p. 2355-9
- Farmaco. Ed.Sci, (1972), vol. 27(9) p. 731-43
- USP 4.595.765
- J. Heterocycl. Chem. (1987), vol. 24(4) p. 941-3
- DE 3.707.088
- EP 0.188.094
- J. Indian Chem. Soc. (1969), vol. 46(1) p. 31-8
- J. Med. Chem. (1986), vol. 29(8) p. 1239-40
- Monatsh. Chem. 68, vol. 99(6) p. 2349-58
- USP 4.672.116
- J. Heterocycl. Chem. (1986), vol. 23(6) p. 1821-8

Les préparations des produits de formule (VII) peuvent également être retrouvées dans les Beilstein.

### EXEMPLE 1 : 3,4-diméthoxy 9-formamido 9,10-dihydrophénanthrène

A une solution de 4,53 g de 9-amino 3,4-diméthoxy 9,10-dihydrophénanthrène (décrit dans J. Med. Chem. 1975, 18(1), 108-10) dans 20 cm³ de benzène anhydre on ajoute une solution de 2,2 g d'anhydride formylacétique dans 20 cm³ de benzène. Après une heure d'agitation à température ambiante on verse dans une solution saturée de bicarbonate de sodium. On ajoute de l'acétate d'éthyle et décante. On lave à l'eau et à l'eau salée, sèche sur sulfate de magnésium et évapore à sec. On obtient 4,8 g de produit cristallisé fondant à 139°C.

Le produit est redissous dans 25 cm³ d'acétate d'éthyle au reflux. On amorce la cristallisation, laisse refroidir puis glace deux heures. On filtre, rince à l'acétate d'éthyle et obtient 3,556 g de produit attendu. F = 139-140°C.

Par évaporation des liqueurs-mères on obtient 1,2 g de produit cristallisé que l'on reprend dans un mélange benzèneacétate d'éthyle (7:3) puis chromatographie sur silice (éluant : benzène-acétate d'éthyle (7:3)) et obtient 656 mg de produit supplémentaire (F = 144-145°C), soit au total 4,21 g de produit.

On obtient un échantillon pur pour analyse en recristallisant 100 mg de produit dans l'acétate d'éthyle au reflux. On obtient ainsi 69 mg de produit purifié. F = 144-145°C.

### Spectre IR (CHCl₃)

NH à 3428 cm⁻¹
Carbonyle complexe 1688-1683 cm⁻¹
Régions aromatiques + NH déformations 1495 cm⁻¹

### Spectre UV

Infl. 230 nm E'₁ = 693 epsilon = 19600
Infl. 260 nm E'₁ = 524
Max. 268 nm E'₁ = 635 epsilon = 18000
Infl. 277 nm E'₁ = 473
Infl. 298 nm E'₁ = 87 epsilon = 2460

### EXEMPLE 2 : 3,4-diméthoxy 9-méthylamino 9,10-dihydrophénanthrène (chlorhydrate)

On ajoute en 20 minutes une solution de 5,35 g de chlorure d'aluminium dans 30 cm³ de tétrahydrofuranne anhydre à une suspension de 1,54 g d'hydrure de lithium aluminium dans 70 cm³ de tétrahydrofuranne anhydre. On agite 15 minutes et ajoute ensuite 3,834 g de 3,4-diméthoxy 9-formamido 9,10-dihydrophénanthrène obtenu à l'exemple 1 en suspension dans 30 cm³ de tétrahydrofuranne. On agite pendant 5 heures et demie.

On verse dans l'eau glacée additionnée de soude, ajoute de l'acétate d'éthyle agite et filtre. On décante, lave à l'eau puis à l'eau salée, sèche sur sulfate de magnésium et évapore les solvants.

On obtient 3,7 g de résine que l'on reprend dans l'acétate d'éthyle à 3 % de triéthylamine et chromatographie sur silice en éluant avec le même mélange.

On recueille 3,31 g de 3,4-diméthoxy 9-méthylamino 9,10-dihydrophénanthrène sous forme de résine.

On obtient le chlorhydrate en opérant comme suit : la résine est dissoute dans 10 cm³ d'acétate d'éthyle et ajoute de l'acétate d'éthyle saturé de gaz chlorhydrique jusqu'à pH1. Le chlorhydrate critallise. On laisse agiter une heure puis évapore à sec. On obtient 3,6 g de chlorhydrate F = 265°C environ.

On obtient un échantillon pour analyse en dissolvant 200 mg de chlorhydrate dans 2 cm³ d'éthanol. On filtre à chaud, laisse refroidir, amorce la cristallisation et glace une heure. Après filtrage et rinçage, on obtient 69 mg de produit purifié. F = 265°C.

### Spectre IR (CHCl₃)

Aromatiques 1606-1572 cm⁻¹
Aromatiques + déformations NH₂ 1495 cm⁻¹
Absorption région OH, NH.

### Spectre UV (EtOH)

Infl. 230 nm E'₁ = 540
Max. 269 nm E'₁ = 564 epsilon = 17200
Infl. 290 nm E'₁ = 172
Infl. 305 nm E'₁ = 75

### EXEMPLE 3 : 3,4-dihydroxy 9-méthylamino 9,10-dihydrophénanthrène (bromhydrate)

On chauffe au reflux pendant une heure sous azote 3,4 g de chlorhydrate de 3,4-diméthoxy 9-méthylamino 9,10-dihydrophénanthrène obtenu à l'exemple 2 dans 25 cm³ d'acide bromhydrique à 66 %.

On laisse refroidir le produit cristallisé et filtre sous vide. On rince à l'isopropanol, sèche en présence de P₂0₅. On obtient 3,157 g de bromhydrate de 3,4-dihydroxy 9-méthylamino 9,10-dihydrophénanthrène attendu. F = 245°C.

Le produit est redissous dans 20 cm³ d'éthanol au reflux, on filtre à chaud, concentre au demi jusqu'à cristallisation et laisse refroidir ; on filtre, rince à l'éthanol et obtient 1,737 g de produit purifié. F = 245°C.

### Spectre IR (nujol)

Aromatique + déformation NH₂ = 1632 cm⁻¹, 1600 cm⁻¹,
1578 cm⁻¹, 1509 cm⁻¹
Région NH, OH associée absorption à 3162 cm⁻¹

### Spectre UV (éthanol)

Infl. 216 nm E'₁ = 855
Infl. 270 nm E'₁ = 468
Max. 274 nm E'₁ = 502 epsilon = 16200
Infl. 285 nm E'₁ = 343
Max. 313,5 nm E'₁ = 115 epsilon = 3700

### Tests pharmacologiques

### 1 - Mesure de l'inhibition de la tyrosinekinase du récepteur de l'EGF (Epidermal Growth Factor).

On utilise des membranes de cellules A431 (ATCC CRL 1555) comme source de récepteurs de l'EGF. Cette lignée cellulaire exprime à sa surface un grand nombre de récepteur de l'EGF qui possèdent une activité tyrosinekinase.

Ces cellules sont préincubées avec ou sans EGF (1000 nM) pendant 15 minutes et ajoutées au tampon HEPES (acide N-2-hydroxyéthyl-piperazine N'-2-éthanesulfonique) correspondant (avec ou sans EGP) et contenant des ions Mg²⁺ (10 mM) et Mn²⁺ (2 mM) 0,2 % de triton X-100, de l'orthovanadate de sodium (20 micromoles) et l'inhibiteur.

Des échantillons contenant ou ne contenant pas le substrat poly(Glu, Ala, Tyr 6 : 3 : 1) sont préparés. On initie la réaction par addition de [gamma³²P] ATP (32 micro-moles). Après incubation pendant 15 minutes à 30°C les échantillons sont précipités à l'aide d'acide chloroacétique à 10 %, filtrés sur membrane millipore et le ³²P incorporé est mesuré avec un compteur de scintillation liquide.

Les résultats sont exprimés en CI₅₀, c'est-à-dire la concentration de substance qu'inhibe 50 % de l'activité de l'enzyme, déterminée par une série de dilutions commençant à 51 microgrammes/ml dans les échantillons contenant le substrat et l'EGF.

On obtient les résultats suivants :
Produit A : 0,6 microgrammes/ml.
Produit de l'exemple 3 : 0,2 microgrammes/ml.

### 2 - Mesure de l'inhibition de la protéinekinase dépendante du 3',5'-AMP cyclique.

La sous-unité catalytique de la protéinekinase dépendante du 3',5'-AMP cyclique a été reconstituée comme indiqué par SIGMA ^{R} . L'activité enzymatique a été mesurée à l'aide de KEMPTID ^{R} (SIGMA ^{R}) comme substrat (KEMPTID - Leu-Arg-Arg-Ala-Ser-Leu-Gly).

L'inhibiteur est préincubé à pH6,9 avec l'enzyme, le substrat (190 micromoles), Mg⁺² (5 mM), 0,25 mg/ml de BSA (Bovine Sérum Albumine) et 3,75 mM de mercaptoéthanol dans 50 mM d'acide 4-morpholinopropanesulfonique.

La réaction est initiée à l'aide de gamma³²P ATP (40 micromoles). Après 15 minutes à 30°C une aliquote est placée sur un papier échangeur d'ion (2 x 2 cm³ ; WHATMAN ^{R}) plongé dans de l'acide phosphorique (75 mM), lavé, séché et l'incorporation de ³²P est mesurée à l'aide d'un compteur de scintillation liquide.

Les résultats sont exprimés en pourcentage d'inhibition de l'activité enzymatique à une concentration d'inhibiteur de 40 microgrammes/ml.

On obtient les résultats suivants :
Produit A : 19 %.
Produit de l'exemple 3 : 18 %.

Dans les tests ci-dessus, le produit A est le 3,4-dihydroxy 9-diméthylamino 9,10-dihydrophénanthrène décrit dans J. Med. Chem. 1975, 18(1), 108-10.

## Revendications

1. Utilisation des produits de formule générale (I) : 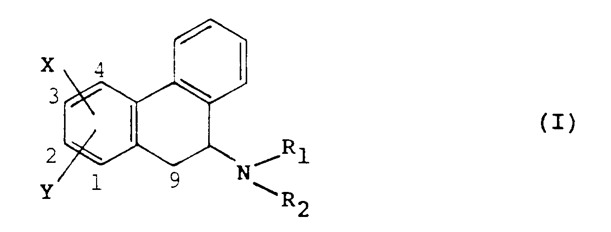 dans laquelle X et Y, identiques ou différents sont choisis parmi les atomes d'hydrogène et les radicaux hydroxy et alkyloxy ayant de 1 à 4 atomes de carbone et R₁ et R₂ identiques ou différents sont choisis parmi les atomes d'hydrogène, les radicaux alkyle, alkényle ou alkynyle ayant au plus 4 atomes de carbone, les radicaux arylalkyle et acyle, ou bien R₁ et R₂ forment ensemble avec l'atome d'azote auquel ils sont liés un hétérocycle à cinq ou six chaînons pouvant comporter un second hétéroatome choisi parmi les atomes d'azote, d'oxygène ou de soufre, éventuellement substitué ainsi que leurs sels d'addition avec les acides minéraux ou organiques pour la préparation d'un médicament anti-tumoral.

2. Utilisation selon la revendication 1 des produits de formule générale (Iₐ) : 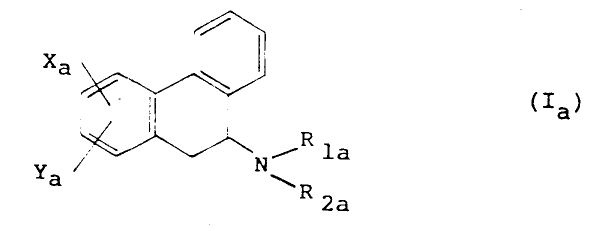 dans laquelle Xₐ et Yₐ sont tels que soit ils représentent chacun un radical hydroxy, soit l'un représente un radical hydroxy et l'autre un atome d'hydrogène,
R₁ₐ et R₂ₐ sont choisis parmi l'hydrogène, les radicaux alkyle ayant de 1 à 4 atomes de carbone, de préférence le méthyle et les radicaux acyle, de préférence formyle, acétyle ou benzoyle, étant entendu qu'ils ne peuvent pas être tous les deux hydrogène, ainsi que leurs sels d'addition avec les acides minéraux ou organiques, de préférence les chlorhydrates, bromhydrates et acétates, pour la préparation d'un médicament anti-tumoral.

3. Utilisation selon l'une des revendications 1 ou 2 de l'un des produits suivants :
- 3,4-dihydroxy 9-méthylamino 9,10-dihydrophénanthrène,
- 3,4-dihydroxy 9-diméthylamino 9,10-dihydrophénanthrène,
et leurs sels d'addition avec les acides minéraux ou organiques, pour la préparation d'un médicament anti-tumoral.

4. A titre de médicaments les produits de formule générale (I') : 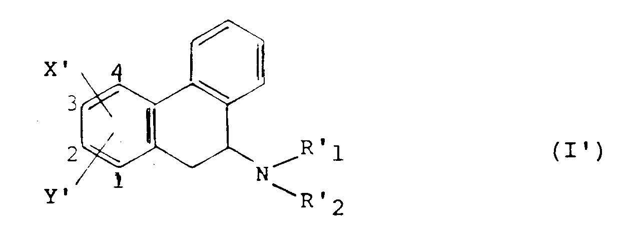 dans laquelle X' et Y', identiques ou différents sont choisis parmi les atomes d'hydrogènes et les radicaux hydroxy et alkyloxy ayant de 1 à 4 atomes de carbone et R'₁ et R'₂ identiques ou différents sont choisis parmi les atomes d'hydrogène, les radicaux alkyle, alkényle ou alkynyle ayant au plus 4 atomes de carbone, les radicaux arylalkyle et acyle, ou bien R'₁ et R'₂ forment ensemble avec l'atome d'azote auquel ils sont liés un hétérocycle à cinq ou six chaînons pouvant comporter un second hétéroatome choisi parmi les atomes d'azote, d'oxygène ou de soufre, éventuellement substitué, étant entendu que :
a) lorsque X' et Y' représentent chacun un radical hydroxy en positions 3 et 4, R'₁ et R'₂ ne peuvent pas représenter chacun un radical méthyle,
b) lorsque X' et Y' représentent chacun un radical hydroxy ou méthoxy en position 2 et 3, R'₁ et R'₂ ne peuvent pas représenter chacun un atome d'hydrogène,
c) lorsque X' et Y' représentent chacun un radical méthoxy en position 2 et 3, R'₁ et R'₂ ne peuvent pas représenter l'un un atome d'hydrogène et l'autre un radical benzoyle,
ainsi que les sels d'addition avec les acides minéraux ou organiques.

5. A titre de médicament, le 3,4-dihydroxy 9-méthylamino 9,10-dihydrophénanthrène et ses sels d'addition avec les acides minéraux ou organiques.

6. Les compositions pharmaceutiques contenant à titre de principe actif l'un au moins des médicaments tels que définis par la revendication 4 ou 5.

7. Les produits de formule générale (I'') : 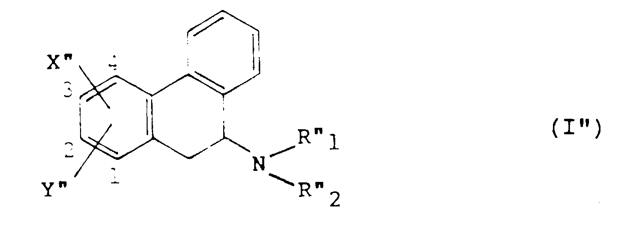 dans laquelle X'' et Y'', identiques ou différents sont choisis parmi les atomes d'hydrogène et les radicaux hydroxy et alkyloxy ayant de 1 à 4 atomes de carbone et R''₁ et R''₂ identiques ou différents sont choisis parmi les atomes d'hydrogène, les radicaux alkyle, alkényle ou alkynyle ayant au plus 4 atomes de carbone, les radicaux arylalkyle et acyle, ou bien
R''₁ et R''₂ forment ensemble avec l'atome d'azote auquel ils sont liés un hétérocycle à cinq ou six chaînons pouvant comporter un second hétéroatome choisi parmi les atomes d'azote, d'oxygène ou de soufre, éventuellement substitué, étant entendu que :
a) lorsque X'' et Y'' représentent chacun un radical hydroxy ou méthoxy en positions 3 et 4, R''₁ et R''₂ ne peuvent pas représenter chacun un radical méthyle,
b) lorsque X'' et Y'' représentent chacun un radical méthoxy en positions 3 et 4, R''₁ et R''₂ ne peuvent pas représenter chacun un atome d'hydrogène,
c) lorsque X'' et Y'' représentent chacun un radical hydroxy ou méthoxy en positions 2 et 3, R''₁ et R''₂ ne peuvent pas représenter chacun un atome d'hydrogène,
d) lorsque X'' et Y'' représentent chacun un radical méthoxy en positions 2 et 3, R''₁ et R''₂ ne peuvent pas représenter l'un un atome d'hydrogène et l'autre un radical benzoyle,
e) lorsque X'' et Y'' représentent chacun un atome d'hydrogène R''₁ et R''₂ sont tels que :
i) R''₁ et R''₂ ne peuvent pas représenter chacun un atome d'hydrogène ou un radical méthyle ou éthyle,
ii) l'un de R''₁ et R''₂ ne peut pas représenter un atome d'hydrogène lorsque l'autre représente un radical méthyle, éthyle, allyle, n-propyle, isopropyle, tert-butyle, benzyle ou phényl-éthyle.
ainsi que leurs sels d'addition avec les acides minéraux ou organiques.

8. Le produit de formule (I'') telle que définie à la revendication 7 répondant à la formule suivante :
- 3,4-dihydroxy 9-méthylamino 9,10-dihydrophénanthrène et ses sels d'addition avec les acides minéraux ou organiques.

9. Procédé de préparation des produits de formule (I'') telle que définie à l'une des revendications 7 ou 8, caractérisé en ce que l'on fait agir, sur un produit de formule (I''ₐ) : 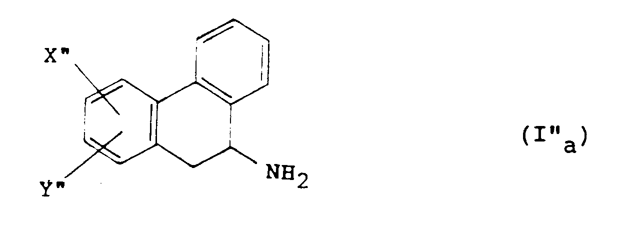 dans laquelle X'' et Y'' ont la signification indiquée à la revendication 7,
soit pour préparer un produit de formule (I''_{b}) correspondant à un produit de formule (I'') dans laquelle l'un de R''₁ et R''₂ est différent d'hydrogène et l'autre représente un atome d'hydrogène,
ou bien un équivalent d'un produit de formule R₁₂Hal dans laquelle R₁₂ représente le radical R''₁ ou R''₂ et Hal représente un atome d'halogène,
ou bien un équivalent d'un produit de formule 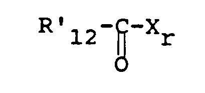 dans laquelle R'₁₂ représente le radical R''₁ ou R''₂ dans lequel on a retranché un atome de carbone et Xᵣ représente un groupement réactif, et soumet éventuellement le produit obtenu à une réduction,
soit pour préparer un produit de formule (I''_{c}) correspondant à un produit de formule (I'') dans laquelle R''₁ et R''₂ sont identiques et ne représentent pas un atome d'hydrogène,
ou bien deux équivalents d'un produit de formule R₁₂Hal dans laquelle R₁₂ et Hal ont la signification précédente,
ou bien deux équivalents d'un produit de formule 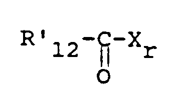 dans laquelle R'₁₂ et Xᵣ ont la signification précédente et soumet éventuellement le produit obtenu à une réduction,
soit pour préparer un produit de formule (I''_{d}) correspondant à un produit de formule (I'') dans laquelle R''₁ et R''₂ sont différents et ne représentent pas un atome d'hydrogène,
ou bien un équivalent d'un produit de formule R₁Hal suivi d'un équivalent d'un produit de formule R₂Hal,
ou bien un équivalent d'un produit de formule 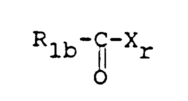 dans laquelle R_{1b} représente le radical R''₁ dans lequel on a retranché un atome de carbone suivi soit d'un équivalent d'un produit de formule R₂Hal, soit d'un équivalent d'un produit de formule 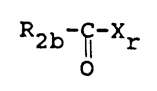 dans laquelle R_{2b} représente le radical R''₂ dans lequel on a retranché un atome de carbone et que dans les deux cas on soumet éventuellement le produit obtenu à une réduction,
soit pour préparer un produit de formule (I''ₑ) correspondant à un produit de formule (I'') dans laquelle R''₁ et R''₂ forment ensemble avec l'atome d'azote auquel ils sont liés un hétérocycle à cinq ou six chaînons pouvant comporter un second hétéroatome choisi parmi les atomes d'azote, d'oxygène ou de soufre éventuellement substitué,
ou bien un produit de formule :
Hal-(CH₂)ₘ-A-(CH₂)ₙ-Hal
dans laquelle m et n sont des entiers différents de zéro choisis de telle façon que leur somme soit égale à 3 ou 4 et A représente un radical méthylène, un atome de soufre, d'oxygène ou d'azote éventuellement substitué,
ou bien un produit de formule : 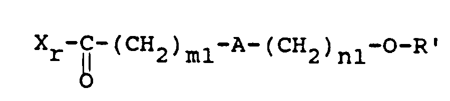 dans laquelle Xᵣ et A ont la signification précédente, m1 et n1 sont des entiers différents de zéro choisis de telle façon que leur somme soit égale à 2 ou 3 et R' représente un groupement protecteur du radical hydroxy, soumet le produit obtenu à une réaction de déprotection dudit radical hydroxy puis transforme ledit radical hydroxy, ou bien en un atome d'halogène ou bien en un dérivé réactif puis soumet l'un ou l'autre des produits obtenus à une réaction de cyclisation et enfin à une réaction de réduction,
et si désiré transforme par hydrolyse les produits de formule (I'') obtenus dans lesquels l'un au moins de X'' ou Y'' représente un radical alkyloxy en produit correspondant dans lequel X'' et Y'' représentent un radical hydroxy et si désiré transforme les produits de formule (I'') en leurs sels d'acides minéraux ou organiques.

## Claims

1. Use of the products of general formula (I): 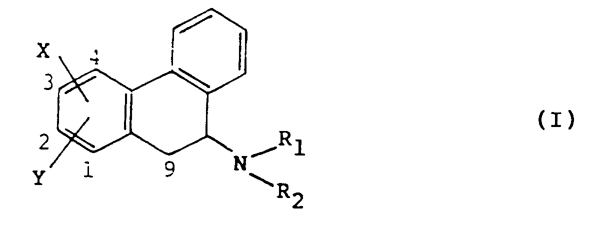 in which X and Y, identical or different, are chosen from hydrogen atoms and hydroxy and alkyloxy radicals having 1 to 4 carbon atoms and R₁ and R₂, identical or different, are chosen from hydrogen atoms, alkyl, alkenyl or alkynyl radicals having at most 4 carbon atoms, arylalkyl and acyl radicals, or R₁ and R₂ form together with the nitrogen atom to which they are linked a heterocycle with five or six members which can contain a second heteroatom chosen from nitrogen, oxygen or sulphur atoms, optionally substituted, as well as their addition salts with mineral or organic acids for the preparation of an anti-tumour medicament.

2. Use according to claim 1 of the products of general formula (Iₐ): 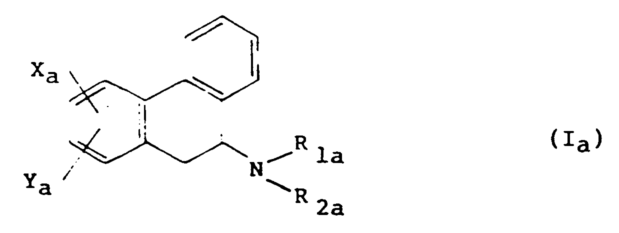 in which Xₐ and Yₐ are such that either they each represent a hydroxy radical, or one represents a hydroxy radical and the other a hydrogen atom,
R₁ₐ and R₂ₐ are chosen from hydrogen, alkyl radicals having 1 to 4 carbon atoms, preferably methyl and acyl radicals, preferably formyl, acetyl or benzoyl, it being understood that they cannot both be hydrogen, as well as their addition salts with mineral or organic acids, preferably hydrochlorides, hydrobromides and acetates, for the preparation of an anti-tumour medicament.

3. Use according to one of claims 1 or 2 of one of the following products:
- 3,4-dihydroxy 9-methylamino 9,10-dihydrophenanthrene,
- 3,4-dihydroxy 9-dimethylamino 9,10-dihydrophenanthrene,
and their addition salts with mineral or organic acids, for the preparation of an anti-tumour medicament.

4. As medicaments, the products of general formula (I'): 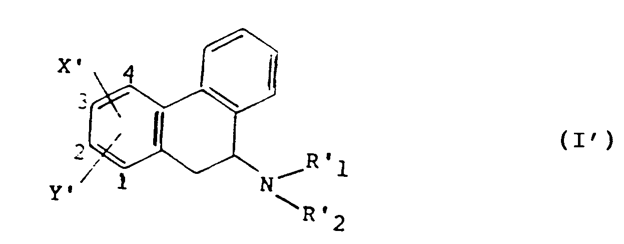 in which X' and Y', identical or different, are chosen from hydrogen atoms and hydroxy and alkyloxy radicals having 1 to 4 carbon atoms and R'₁ and R'₂, identical or different, are chosen from hydrogen atoms, alkyl, alkenyl or alkynyl radicals having at most 4 carbon atoms, arylalkyl and acyl radicals, or R'₁ and R'₂ form together with the nitrogen atom to which they are linked a heterocycle with five or six members which can contain a second heteroatom chosen from nitrogen, oxygen or sulphur atoms, optionally substituted, it being understood that:
a) when X' and Y' each represent a hydroxy radical in positions 3 and 4, R'₁ and R'₂ cannot each represent a methyl radical,
b) when X' and Y' each represent a hydroxy or methoxy radical in positions 2 and 3, R'₁ and R'₂ cannot each represent a hydrogen atom,
c) when X' and Y' each represent a methoxy radical in positions 2 and 3, one of R'₁ and R'₂ cannot represent a hydrogen atom and the other cannot represent a benzoyl radical,
as well as the addition salts with the mineral or organic acids.

5. As a medicament, 3,4-dihydroxy 9-methylamino 9,10-dihydrophenanthrene and its addition salts with mineral or organic acids.

6. The pharmaceutical compositions containing as active ingredient at least one of the medicaments as defined by claim 4 or 5.

7. The products of general formula (I''): 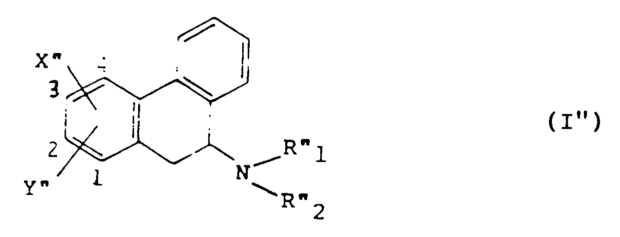 in which X'' and Y'', identical or different, are chosen from hydrogen atoms and hydroxy and alkyloxy radicals having 1 to 4 carbon atoms and R''₁ and R''₂, identical or different, are chosen from hydrogen atoms, alkyl, alkenyl or alkynyl radicals having at most 4 carbon atoms, arylalkyl and acyl radicals, or R''₁ and R''₂ form together with the nitrogen atom to which they are linked a heterocycle with five or six members which can contain a second heteroatom chosen from nitrogen, oxygen or sulphur atoms, optionally substituted, it being understood that:
a) when X'' and Y'' each represent a hydroxy or methoxy radical in positions 3 and 4, R''₁ and R''₂ cannot each represent a methyl radical
b) when X'' and Y'' each represent a methoxy radical in positions 3 and 4, R''₁ and R''₂ cannot each represent a hydrogen atom,
c) when X'' and Y'' each represent a hydroxy or methoxy radical in positions 2 and 3, R''₁ and R''₂ cannot each represent a hydrogen atom,
d) when X'' and Y'' each represent a methoxy radical in positions 2 and 3, one of R''₁ and R''₂ cannot represent a hydrogen atom and the other cannot represent a benzoyl radical,
e) when X'' and Y'' each represent a hydrogen atom, R''₁ and R''₂ are such that:
i) R''₁ and R''₂ cannot each represent a hydrogen atom or a methyl or ethyl radical,
ii) one of R''₁ and R''₂ cannot represent a hydrogen atom when the other represents a methyl, ethyl, allyl, n-propyl, isopropyl, tert-butyl, benzyl or phenyl-ethyl radical,
as well as their addition salts with mineral or organic acids.

8. The product of formula (I'') as defined in claim 7 corresponding to the following formula:
- 3,4-dihydroxy 9-methylamino 9,10-dihydrophenanthrene and its addition salts with mineral or organic acids.

9. Preparation process for products of formula (I'') as defined in one of claims 7 or 8, characterized in that one 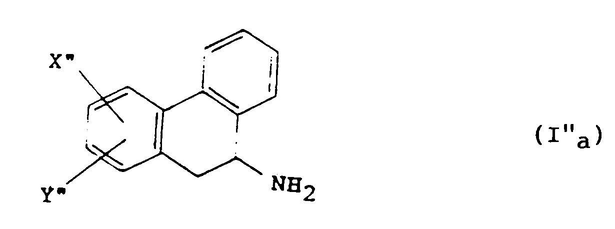 reacts, on a product of formula (I''ₐ): in which X'' and Y'' have the meaning indicated in claim 7,
either to prepare a product of formula (I''_{b}) corresponding to a product of formula (I'') in which one of R''₁ and R''₂ is different from hydrogen and the other represents a hydrogen atom,
either an equivalent of a product of formula R₁₂Hal in which R₁₂ represents the R''₁ or R''₂ radical and Hal represents a halogen atom,
or an equivalent of a product of formula 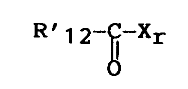 in which R'₁₂ represents the R''₁ or R''₂ radical from which a carbon atom has been removed and Xᵣ represents a reactive group, and the product obtained is optionally subjected to a reduction,
or to prepare a product of formula (I''_{c}) corresponding to a product of formula (I'') in which R''₁ and R''₂ are identical and do not represent a hydrogen atom,
either two equivalents of a product of formula R₁₂Hal in which R₁₂ and Hal have the previous meaning,
or two equivalents of a product of formula 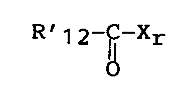 in which R'₁₂ and Xᵣ have the previous meaning
and the product obtained is optionally subjected to a reduction,
or to prepare a product of formula (I''_{d}) corresponding to a product of formula (I'') in which R''₁ and R''₂ are different and do not represent a hydrogen atom,
either an equivalent of a product of formula R₁Hal followed by an equivalent of a product of formula R₂Hal,
or an equivalent of a product of formula 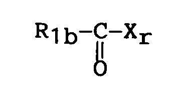 in which R_{1b} represents the R''₁ radical from which a carbon atom has been removed followed either by an equivalent of a product of formula R₂Hal, or of an equivalent of a product of formula 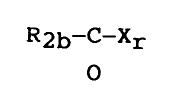 in which R_{2b} represents the R''₂ radical from which a carbon atom has been removed and in both cases the product obtained is optionally subjected to a reduction,
or to prepare a product of formula (I''ₑ) corresponding to a product of formula (I'') in which R''₁ and R''₂ form together with the nitrogen atom to which they are linked a heterocycle with five or six members which can contain a second heteroatom chosen from nitrogen, oxygen or sulphur atoms, optionally substituted,
either a product of formula:
Hal-(CH₂)ₘ-A-(CH₂)ₙ-Hal
in which m and n are integers different from zero chosen in such a way that their sum is equal to 3 or 4 and A represents a methylene radical, an sulphur, oxygen or nitrogen atom, optionally substituted,
or a product of formula: 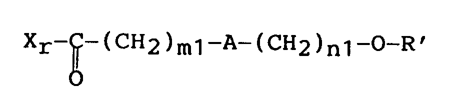 in which Xᵣ and A have the previous meaning, m1 and n1 are integers different from zero chosen in such a way that their sum is equal to 2 or 3 and R' represents a protective group of the hydroxy radical, the product obtained is subject to a deprotection reaction of the said hydroxy radical then the said hydroxy radical is converted, either into a halogen atom or into a reactive derivative then one or other of the products obtained is subjected to a cyclization reaction and finally to a reduction reaction,
and if desired the products of formula (I'') obtained in which at least one of X'' or Y'' represents an alkyloxy radical is converted by hydrolysis into a corresponding product in which X'' and Y'' represent a hydroxy radical and if desired the products of formula (I'') are converted into their mineral or organic acid salts.

## Patentansprüche

1. Verwendung von Produkten der allgemeinen Formel (I) 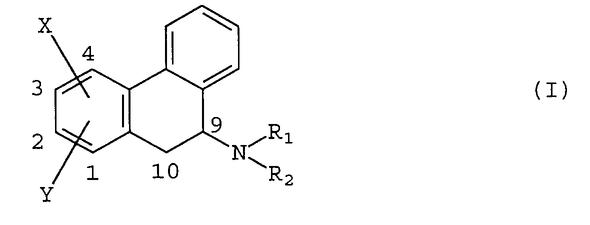 in der X und Y, gleich oder verschieden, unter den Wasserstoffatomen und den Resten Hydroxy und Alkyloxy mit 1 bis 4 Kohlenstoffatomen, und R₁ und R₂, gleich oder verschieden, unter den Wasserstoffatomen, den Resten Alkyl, Alkenyl oder Alkinyl mit höchstens 4 Kohlenstoffatomen sowie den Resten Arylalkyl und Acyl ausgewählt werden, oder R₁ und R₂ zusammen mit dem Stickstoffatom, an das sie gebunden sind, einen Heterocyclus mit 5 oder 6 Ringgliedern bilden, der auch ein zweites Heteroatom umfassen kann, ausgewählt unter den Atomen von Stickstoff, Sauerstoff oder Schwefel und gegebenenfalls substituiert, sowie ihrer Additionssalze mit Mineralsäuren oder organischen Säuren für die Herstellung eines anti-tumoralen Medikamentes.

2. Verwendung nach Anspruch 1 der Produkte der allgemeinen Formel (Iₐ) 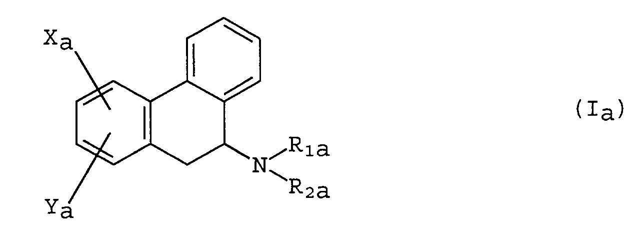 in der Xₐ und Yₐ entweder jeweils einen Hydroxyrest darstellen oder eines von ihnen stellt einen Hydroxyrest dar und das andere ein Wasserstoffatom,
R₁ₐ und R₂ₐ unter Wasserstoff, den Alkylresten mit 1 bis 4 Kohlenstoffatomen, vorzugsweise Methyl, und den Acylresten, vorzugsweise Formyl, Acetyl oder Benzoyl, ausgewählt werden, mit der Maßgabe, daß sie nicht alle beide Wasserstoff sein können, sowie ihrer Additionssalze mit Mineralsäuren oder organischen Säuren, vorzugsweise der Hydrochloride, Hydrobromide und Acetate, für die Herstellung eines anti-tumoralen Medikamentes.

3. Verwendung nach einem der Ansprüche 1 oder 2 eines der folgenden Produkte
- 3,4-Dihydroxy-9-methylamino-9,10-dihydrophenanthren,
- 3,4-Dihydroxy-9-dimethylamino-9,10-dihydrophenanthren,
und ihrer Additionssalze mit Mineralsäuren oder organischen Säuren für die Herstellung eines anti-tumoralen Medikamentes.

4. Als Medikamente die Produkte der allgemeinen Formel (I') 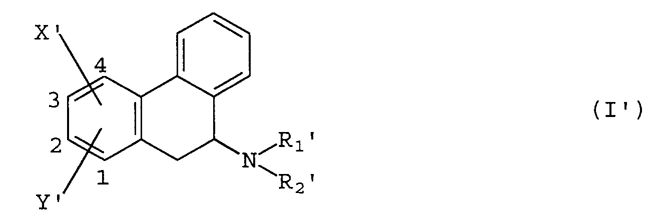 in der X' und Y', gleich oder verschieden, unter den Wasserstoffatomen und den Resten Hydroxy und Alkyloxy mit 1 bis 4 Kohlenstoffatomen, und R₁' und R₂', gleich oder verschieden, unter den Wasserstoffatomen, den Resten Alkyl, Alkenyl oder Alkinyl mit höchstens 4 Kohlenstoffatomen sowie den Resten Arylalkyl und Acyl ausgewählt werden, oder R₁' und R₂' zusammen mit dem Stickstoffatom, an das sie gebunden sind, einen Heterocyclus mit 5 oder 6 Ringgliedern bilden, der auch ein zweites Heteroatom umfassen kann, ausgewählt unter den Atomen von Stickstoff, Sauerstoff oder Schwefel und gegebenenfalls substituiert, mit der Maßgabe, daß
a) wenn X' und Y' jeweils einen Hydroxyrest in Position 3 und 4 darstellen, R₁' und R₂' nicht jeweils einen Methylrest bedeuten können,
b) wenn X' und Y' jeweils einen Hydroxyrest oder Methoxyrest in Position 2 und 3 darstellen, R₁' und R₂' nicht jeweils ein Wasserstoffatom bedeuten können,
c) wenn X' und Y' jeweils einen Methoxyrest in Position 2 und 3 darstellen, R₁' und R₂' nicht eines ein Wasserstoffatom und das andere einen Benzoylrest bedeuten können,
sowie die Additionssalze mit Mineralsäuren oder organischen Säuren.

5. Als Medikament das 3,4-Dihydroxy-9-methylamino-9,10-dihydrophenanthren und seine Additionssalze mit Mineralsäuren oder organischen Säuren.

6. Pharmazeutische Zusammensetzungen, die als Wirkstoff mindestens eines der in Anspruch 4 oder 5 definierten Medikamente enthalten.

7. Die Produkte der allgemeinen Formel (I'') 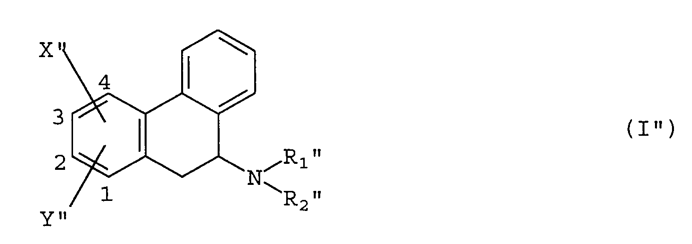 in der X'' und Y'', gleich oder verschieden, unter den Wasserstoffatomen und den Resten Hydroxy und Alkyloxy mit 1 bis 4 Kohlenstoffatomen, und R₁'' und R₂'', gleich oder verschieden, unter den Wasserstoffatomen, den Resten Alkyl, Alkenyl oder Alkinyl mit höchstens 4 Kohlenstoffatomen sowie den Resten Arylalkyl und Acyl ausgewählt werden, oder R₁'' und R₂'' zusammen mit dem Stickstoffatom, an das sie gebunden sind, einen Heterocyclus mit 5 oder 6 Ringgliedern bilden, der auch ein zweites Heteroatom umfassen kann, ausgewählt unter den Atomen von Stickstoff, Sauerstoff oder Schwefel und gegebenenfalls substituiert, mit der Maßgabe, daß
a) wenn X'' und Y'' jeweils einen Hydroxyrest oder Methoxyrest in Position 3 und 4 darstellen, R₁'' und R₂'' nicht jeweils einen Methylrest bedeuten können,
b) wenn X'' und Y'' jeweils einen Methoxyrest in Position 3 und 4 darstellen, R₁'' und R₂'' nicht jeweils ein Wasserstoffatom bedeuten können,
c) wenn X'' und Y'' jeweils einen Hydroxyrest oder Methoxyrest in Position 2 und 3 darstellen, R₁'' und R₂'' nicht jeweils ein Wasserstoffatom bedeuten können,
d) wenn X'' und Y'' jeweils einen Methoxyrest in Position 2 und 3 darstellen, R₁'' und R₂'' nicht eines ein Wasserstoffatom und das andere einen Benzoylrest bedeuten können,
e) wenn X'' und Y'' jeweils ein Wasserstoffatom darstellen, bedeuten R₁'' und R₂'' :
i) R₁'' und R₂'' können nicht jeweils ein Wasserstoffatom oder einen der Reste Methyl oder Ethyl bedeuten,
ii) eines von R₁'' und R₂'' kann nicht ein Wasserstoffatom darstellen, wenn das andere einen der Reste Methyl, Ethyl, Allyl, n-Propyl, Isopropyl, tert.-Butyl, Benzyl oder Phenylethyl bedeutet,
sowie ihre Additionssalze mit Mineralsäuren oder organischen Säuren.

8. Das Produkt der Formel (I''), wie in Anspruch 7 definiert, das der folgenden Formel entspricht:
- 3,4-Dihydroxy-9-methylamino-9,10-dihydrophenanthren und seine Additionssalze mit Mineralsäuren oder organischen Säuren.

9. Verfahren zur Herstellung von Produkten der Formel (I'') wie in einem der Ansprüche 7 oder 8 definiert, dadurch gekennzeichnet, daß man ein Produkt der Formel (Iₐ'') 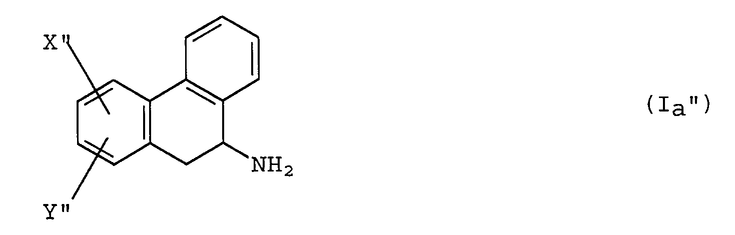 in der X'' und Y'' die in Anspruch 7 angegebene Bedeutung besitzen,
entweder zur Herstellung eines Produktes der Formel (I_{b}''), das einem Produkt der Formel (I'') entspricht, in der eines von R₁'' und R₂'' von Wasserstoff verschieden ist und das andere ein Wasserstoffatom bedeutet, zur Reaktion bringt mit
- einem Äquivalent eines Produktes der Formel R₁₂Hal, in der R₁₂ den Rest R₁'' oder R₂'' darstellt und Hal ein Halogenatom bedeutet, oder mit
- einem Äquivalent eines Produktes der Formel R₁₂'-CO-Xᵣ, in der R₁₂' den Rest R₁'' oder R₂'' darstellt, bei dem man ein Kohlenstoffatom entfernt hat und Xᵣ eine reaktive Gruppe bedeutet, und man gegebenenfalls das erhaltene Produkt einer Reduktion unterzieht,
oder zur Herstellung eines Produktes der Formel (I_{c}''), das einem Produkt der Formel (I'') entspricht, in der R₁'' und R₂'' identisch sind und nicht ein Wasserstoffatom darstellen, zur Reaktion bringt mit
- zwei Äquivalenten eines Produktes der Formel R₁₂Hal, in der R₁₂ und Hal die vorstehend angegebenen Bedeutungen besitzen, oder mit
- zwei Äquivalenten eines Produktes der Formel R₁₂'-CO-Xᵣ, in der R₁₂' und Xᵣ die vorstehend angegebenen Bedeutungen besitzen, und man gegebenenfalls das erhaltene Produkt einer Reduktion unterzieht,
oder zur Herstellung eines Produktes der Formel (I_{d}''), das einem Produkt der Formel (I'') entspricht, in der R₁'' und R₂'' verschieden sind und nicht ein Wasserstoffatom darstellen, zur Reaktion bringt mit
- einem Äquivalent eines Produktes der Formel R₁Hal, gefolgt von einem Äquivalent eines Produktes der Formel R₂Hal, oder mit
- einem Äquivalent eines Produktes der Formel R_{1b}-CO-Xᵣ, in der R_{1b} den Rest R₁'' darstellt, bei dem man ein Kohlenstoffatom entfernt hat, gefolgt entweder von einem Äquivalent eines Produktes der Formel R₂Hal oder einem Äquivalent eines Produktes der Formel R_{2b}-CO-Xᵣ, in der R_{2b} den Rest R₂'' darstellt, bei dem man ein Kohlenstoffatom entfernt hat, und daß man in den zwei Fällen das erhaltene Produkt gegebenenfalls einer Reduktion unterzieht,
oder zur Herstellung eines Produktes der Formel (Iₑ''), das einem Produkt der Formel (I'') entspricht, in der R₁'' und R₂'' zusammen mit dem Stickstoffatom, an das sie gebunden sind, einen Heterocyclus mit 5 oder 6 Ringgliedern bilden, der auch ein zweites Heteroatom umfassen kann, ausgewählt unter den Atomen von Stickstoff, Sauerstoff oder Schwefel und gegebenenfalls substituiert, zur Reaktion bringt mit
- einem Produkt der Formel
Hal-(CH₂)ₘ-A-(CH₂)ₙ-Hal
in der m und n von null verschiedene ganze Zahlen darstellen, die in der Weise gewählt werden, daß ihre Summe gleich 3 oder 4 ist und A einen Methylenrest, ein Schwefel-, Sauerstoff- oder Stickstoffatom bedeutet, gegebenenfalls substituiert, oder mit
- einem Produkt der Formel
Xᵣ-CO-(CH₂)ₘ₁-A-(CH₂)ₙ₁-O-R'
in der Xᵣ und A die vorstehend angegebenen Bedeutungen besitzen, m1 und n1 von null verschiedene ganze Zahlen darstellen, die in der Weise gewählt werden, daß ihre Summe gleich 2 oder 3 ist und R' eine Schutzgruppe für den Hydroxyrest darstellt, und man das erhaltene Produkt einer Reaktion zur Abspaltung der Schutzgruppe von dem genannten Hydroxyrest unterzieht, und man anschließend den genannten Hydroxyrest entweder in ein Halogenatom umwandelt oder in ein reaktives Derivat, wonach man das eine oder andere der erhaltenen Produkte einer Reaktion zur Cyclisierung und schließlich einer Reduktionsreaktion unterzieht,
und man, wenn gewünscht, die erhaltenen Produkte der Formel (I''), in der mindestens eines von X'' oder Y'' einen Alkyloxyrest darstellt, durch Hydrolyse in das entsprechende Produkt umwandelt, in dem X'' und Y'' einen Hydroxyrest bedeuten, und man, wenn gewünscht, die Produkte der Formel (I'') in ihre Salze mit Mineralsäuren oder organischen Säuren umwandelt.
